# EUROPEAN PATENT SPECIFICATION

(11) **EP 2 878 949 B1**
(45) Date of publication and mention of the grant of the patent: **30.08.2017**
(21) Application number: 14193969.4
(22) Date of filing: 20.11.2014
(51) Int. Cl.: G01N 33/497

(54) **Gas concentration measurement apparatus, notification method, and program**
Vorrichtung zur Messung von Gaskonzentrationen, Benachrichtigungsverfahren und Programm
Appareil de mesure de la concentration de gaz, procédé de notification et programme

(30) Priority: 28.11.2013 JP 2013245810
(43) Date of publication of application: 03.06.2015
(73) Proprietor: Tanita Corporation, Tokyo (JP)
(72) Inventor: Sagawa, Kiyoshi, Itabashi-ku, Tokyo (JP); Mochizuki, Kei, Itabashi-ku, Tokyo (JP)
(74) Representative: Grünecker Patent- und Rechtsanwälte PartG mbB

(56) References cited:
- GB-A- 2 435 099
- US-A1- 2010 043 524
- US-B2- 7 919 754

## Description

### BACKGROUND

### Field of the Invention

The present invention relates to a gas concentration measurement apparatus, a notification method, and a program.

### Background

Gas concentration measurement apparatuses are provided with a sensor that outputs, as a result of a chemical reaction, an electrical signal according to the concentration of a gas representing a measurement target. For example, an alcohol sensor provided in an alcohol concentration measurement apparatus outputs, as a result of a chemical reaction, an electrical signal according to the alcohol concentration. Alcohol sensors lose their activation (activity) when poisoned, and the alcohol detection accuracy decreases. In Japanese Patent Application, Publication No. JP2010-223902A, there is disclosed a technique that measures the degradation of an alcohol sensor. GB 2435099 discloses a gas concentration measurement apparatus according to the preamble of claim 1.

### SUMMARY

If the environment in which a gas concentration measurement apparatus is stored is high in a poisoning substance of a gas sensor (for example, in the case of an alcohol sensor, volatile organic compounds, silicon compounds, and the like), poisoning of the gas sensor may progress even when the gas concentration measurement apparatus is not utilized. Therefore, it can be considered to improve the air-tightness of a housing that accommodates the gas sensor in order to prevent the poisoning that occurs due to the environment. That is to say, by improving the air-tightness of the housing, and isolating the gas sensor from the outside air when the gas sensor is not utilized, the gas sensor can be prevented from being poisoned by a poisoning substance present on the exterior of the housing.

However, in a case where the air-tightness of the housing is improved, if the gas sensor is isolated from the outside air while a poisoning substance contained in a subject gas (for example, in the case of an alcohol sensor, sulfur compounds contained in the breath) is deposited on the gas sensor, the poisoning substance becomes confined (contained) in the housing interior, and there is a possibility of degradation of the gas sensor progressing even further. The present invention provides therefore a gas concentration measurement apparatus, a notification method for said apparatus and a computer-readable storage medium stored with a notification program for said apparatus according to independent claims 1, 10 and 14. In accordance with the present invention, it is possible to prevent a gas sensor from degradation due to a poisoning substance being confined in the housing interior.

### BRIEF DESCRIPTION OF THE DRAWINGS

FIG. 1 is a drawing showing external views of an alcohol concentration measurement apparatus according to at least one embodiment.
FIG. 2 is a schematic block diagram showing a configuration of an alcohol concentration measurement apparatus according to a first embodiment.
FIG. 3 is a flowchart showing operation of the alcohol concentration measurement apparatus according to the first embodiment.
FIG. 4 is a drawing showing examples of images that are displayed on a display of the alcohol concentration measurement apparatus according to the first embodiment.
FIG. 5 is a schematic block diagram showing a configuration of an alcohol concentration measurement apparatus according to a second embodiment.
FIG. 6 is a schematic block diagram showing a configuration of an alcohol concentration measurement apparatus according to a third embodiment.
FIG. 7 is a flowchart showing operation of the alcohol concentration measurement apparatus according to the third embodiment.
FIG. 8 is a schematic block diagram showing a configuration of a computer according to at least one embodiment.

### BRIEF DESCRIPTION OF THE REFERENCE NUMERALS

- 1: ALCOHOL CONCENTRATION MEASUREMENT APPARATUS
- 10: HOUSING
- 11: ENGAGING PART
- 12: VENTILATION HOLES
- 13: DISPLAY
- 14: SPEAKER
- 15: OPEN/CLOSE SENSOR
- 16: BREATH SENSOR
- 17: SEMICONDUCTOR ALCOHOL SENSOR
- 18: CONTROL DEVICE
- 20: LID PART
- 181: SENSOR VALUE ACQUISITION PART
- 182: TIMING PART
- 183: MEASURING PART
- 184: NOTIFICATION PART
- 900: COMPUTER
- 901: CPU
- 902: MAIN STORAGE DEVICE
- 903: AUXILIARY STORAGE DEVICE
- 904: INTERFACE

### DESCRIPTION OF THE EMBODIMENTS

### <First Embodiment>

Hereunder an embodiment is described in detail with reference to the drawings.

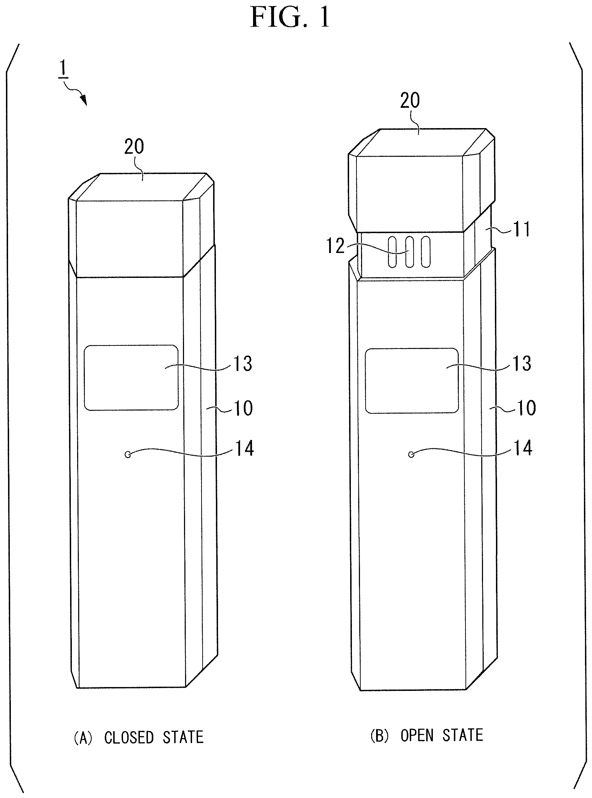

FIG. 1 is a drawing showing external views of an alcohol concentration measurement apparatus 1 according to at least one embodiment. The alcohol concentration measurement apparatus according to the present embodiment is an example of a gas concentration measurement apparatus. In one example, the alcohol concentration measurement apparatus 1 is a portable apparatus (a portable gas sensor, a portable alcohol sensor). For example, the alcohol concentration measurement apparatus 1 is a hand-held size and is configured so as to be capable of being carried or moved along with a person.

The alcohol concentration measurement apparatus 1 is provided with a housing (a main body) 10 and a lid part (a lid member, a cover member, a shield member, a cap member, a member) 20.

FIG. 1(A) shows a state where the lid part 20 of the alcohol concentration measurement apparatus 1 is closed. FIG. 1 (B) shows a state where the lid part 20 of the alcohol concentration measurement apparatus 1 is open.

The housing 10 has a substantially rectangular parallelepiped shape that accommodates a semiconductor alcohol sensor 17 in its interior.

An engaging part 11 that engages with the lid part 20 is provided on one of the longitudinal direction ends of the housing 10. The outer perimeter of the engaging part 11 is smaller than the outer perimeter of the housing 10, and engages with the inner perimeter of the lid part 20. The engaging part 11 is provided with ventilation holes 12 that take outside air into the interior of the housing 10.

The lid part 20 has a box-like shape (a cap-like shape) having an opening on one end. The lid part 20 engages with the engaging part 11, and is configured such that the lid part 20 is slidable from a position that shields the entire engaging part 11, to a position at which the ventilation holes 12 of the engaging part 11 are exposed. In other words, the lid part 20 is configured to be slidable with respect to a part of the housing 10. The lid part 20 is arranged so that the semiconductor alcohol sensor 17 is isolated from the outside air when the lid part 20 is arranged at an closing position and that the semiconductor alcohol sensor 17 is exposed to the outside air when the lid part 20 is arranged at an opening position. In one example, the lid part 20 is arranged to prevent detachment from the housing 10. The housing 10 is configured to prevent detachment of the lid part 20. The interior of the housing 10 is isolated from the outside air by shielding the entire engaging part 11 with the lid part 20, that is to say by closing the lid part 20. The semiconductor alcohol sensor 17 is exposed to the outside air when the lid part 20 is arranged at the opening position. In order to prevent the semiconductor alcohol sensor 17 from being poisoned by the environment, the housing 10 is designed such that the air-tightness of the interior becomes high when the lid part 20 engages with the engaging part 11. The air-tightness of the housing 10 can be increased by using a sealing material such as rubber. However this can also be a cause of degradation of the semiconductor alcohol sensor 17. Alternatively, it is possible to increase the air-tightness by using a metallic frame for the frame of the housing 10, and suppressing the looseness between the lid part 20 and the housing 10.

The housing 10 is provided with a display 13. The display 13 is configured by an LCD (Liquid Crystal Display), or the like. The display 13 displays; whether or not an alcohol concentration measurement can be started, the measured alcohol concentration, a notification prompting exposure of the semiconductor alcohol sensor 17 to the outside air, and the like. Furthermore, the housing 10 is provided with a speaker 14. The speaker 14 outputs a notification of prompting exposure of the semiconductor alcohol sensor 17 to the outside air or the like.

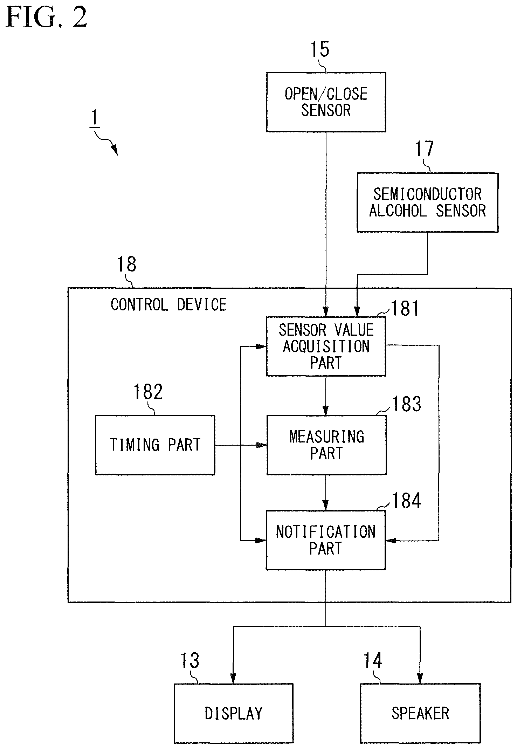

FIG. 2 is a schematic block diagram showing a configuration of the alcohol concentration measurement apparatus 1 according to the first embodiment.

The alcohol concentration measurement apparatus 1 is provided with an open/close sensor 15, the semiconductor alcohol sensor 17, and a control device 18 in the interior of the housing 10.

The open/close sensor 15 detects the opening and closing of the lid part 20, and outputs an electrical signal indicating whether the lid part 20 is open or closed. The open/close sensor 15 can be realized for example by a switch provided on the end of the engaging part 11 that becomes depressed by the lid part 20 when the lid part 20 is closed, and released when the lid part 20 is opened. In one example, the open/close sensor 15 is configured to detect whether the lid part 20 is at the closing position or not.

The semiconductor alcohol sensor 17 converts the alcohol concentration of a breath taken in from the ventilation holes 12 into an electrical signal, and outputs the signal to the control device 18. Specifically, the semiconductor alcohol sensor 17 is provided with a semiconductor (for example, a tin oxide film) whose resistance value changes according to the amount of oxygen in the surroundings, and a heater that heats the semiconductor. When alcohols become adsorbed on the surface of the semiconductor, the amount of oxygen relatively decreases due to a chemical reaction that occurs between the oxygen and alcohol gases. Furthermore, the heater promotes the reaction between the oxygen and alcohol gases by heating the semiconductor. Consequently, the higher the alcohol content in the breath, the more the resistance value of the semiconductor decreases. On the other hand, if poisoning of the semiconductor alcohol sensor 17 occurs as a result of a poisoning substance contained in the outside air or the breath, the sensitivity of the semiconductor toward alcohol deteriorates. That is to say, degradation of the semiconductor alcohol sensor 17 occurs. In the present embodiment, the semiconductor alcohol sensor 17 is an example of a gas sensor (a biological gas sensor).

The control device 18 is provided with a sensor value acquisition part 181, a timing part 182, a measuring part 183, and a notification part 184.

The sensor value acquisition part 181 receives electrical signals from the open/close sensor 15 and the semiconductor alcohol sensor 17, and acquires sensor values based on the electrical signals.

The timing part 182 measures respectively; an elapsed time from the time at which the lid part 20 is opened, an elapsed time from the time at which delivery of a breath onto the semiconductor alcohol sensor 17 occurs (the time at which a breath is blown/supplied to the semiconductor alcohol sensor 17), and an elapsed time from the time at which the alcohol concentration measurement is completed by the measuring part 183.

The measuring part 183 measures the alcohol concentration in a breath based on a sensor value of the semiconductor alcohol sensor 17. In other words, the measuring part 183 is configured to measure a concentration of a gas on the basis of an output signal from the semiconductor alcohol sensor 17. The semiconductor alcohol sensor 17 is configured to output an electrical signal according to a concentration of a gas representing a measurement target.

The notification part 184 displays on the display 13 and further outputs from the speaker 14, whether or not an alcohol concentration measurement can be started, the measured alcohol concentration, and a notification prompting exposure of the semiconductor alcohol sensor 17 to the outside air. In other words, the notification part 184 is configured to issue a notification.

Next, operation of the alcohol concentration measurement apparatus 1 according to the present embodiment is described.

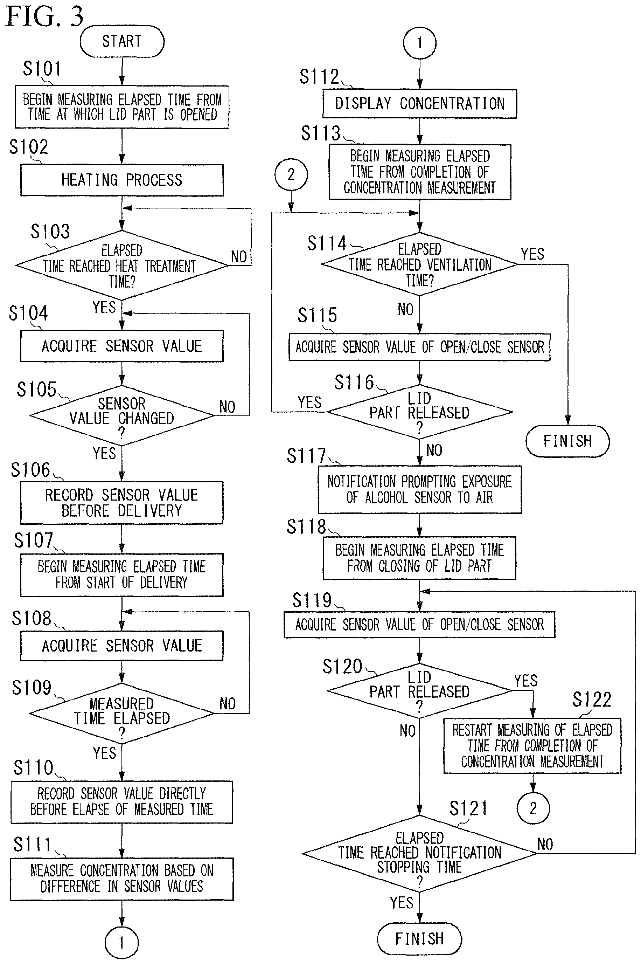

FIG. 3 is a flowchart showing operation of the alcohol concentration measurement apparatus 1 according to the first embodiment.

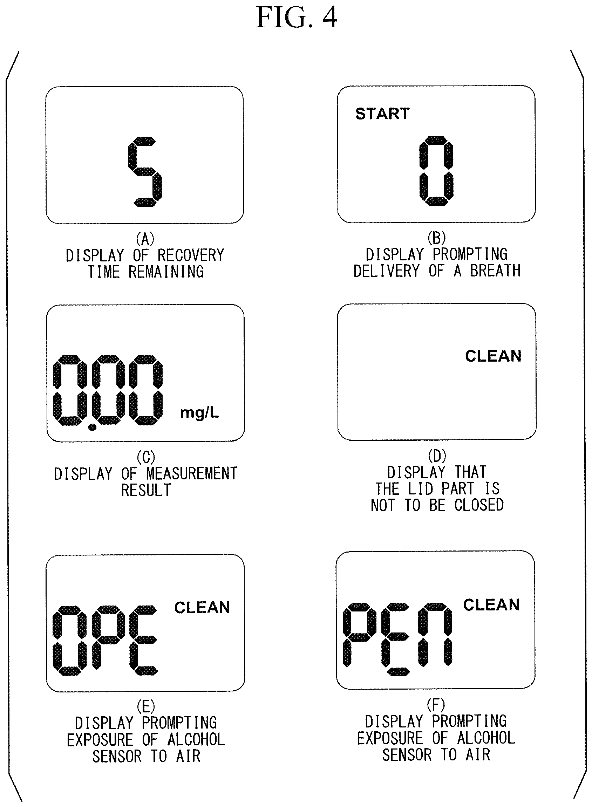

FIG. 4 is a drawing showing examples of images that are displayed on the display 13 of the alcohol concentration measurement apparatus 1 according to the first embodiment.

When a test subject releases the lid part 20 by sliding the lid part 20 in a direction that separates the lid part 20 from the housing 10, the open/close sensor 15 detects the releasing of the lid part 20 and outputs an electrical signal that indicates the releasing of the lid part 20. Consequently, the power of the alcohol concentration measurement apparatus 1 turns on.

When the sensor value acquisition part 181 acquires from the open/close sensor 15, an electrical signal that indicates the releasing of the lid part 20, the timing part 182 begins measuring the elapsed time from the time at which the lid part 20 is opened (step S101). Next, the semiconductor alcohol sensor 17 heats the heater, and starts a heating process with respect to the semiconductor (step S102). The heating process promotes reaction between the oxygen and alcohol gases in the surroundings of the semiconductor, and is also a process carried out in order to remove contaminants deposited on the semiconductor.

Next, the measuring part 183 determines whether or not the elapsed time measured by the timing part 182 has reached a predetermined heat treatment time (for example, 10 seconds) (step S103). If the elapsed time measured by the timing part 182 has not reached the heat treatment time (step S103: NO), the process returns to step S103 and repeats the determination of whether or not the elapsed time has reached the heat treatment time. The alcohol concentration measurement apparatus 1 accepts the delivery of a breath after the elapsed time measured by the timing part 182 reaches the predetermined heat treatment time. That is to say, even if the delivery of a breath occurs before the heat treatment time has passed, the measuring part 183 does not perform an alcohol concentration measurement. Furthermore, until the elapsed time measured by the timing part 182 reaches the heat treatment time, the notification part 184 displays on the display 13 as shown in FIG. 4(A), the time remaining until the elapsed time reaches the heat treatment time.

On the other hand, if the measuring part 183 determines that the elapsed time measured by the timing part 182 has reached the heat treatment time (step S103: YES), the sensor value acquisition part 181 acquires a sensor value from the electrical signal output by the semiconductor alcohol sensor 17 (step S104). The measuring part 183 determines whether or not a change in the sensor value of the semiconductor alcohol sensor 17 acquired by the sensor value acquisition part 181 is equal to or greater than a predetermined threshold (step S105). If a breath has been delivered to the semiconductor alcohol sensor 17, the oxygen concentration changes in the surroundings of the semiconductor of the semiconductor alcohol sensor 17. Consequently, in the present embodiment, the delivery of a breath is determined by the presence of a change in the sensor value of the semiconductor alcohol sensor 17.

If the measuring part 183 determines that there is no change in the sensor value of the semiconductor alcohol sensor 17 (step S105: NO), the process returns to step S104 and awaits the delivery of a breath. Until the delivery of a breath is detected, the notification part 184 prompts the test subject to deliver a breath by displaying the word "START" on the display 13 as shown in FIG. 4(B).

If the measuring part 183 determines that there is a change in the sensor value of the semiconductor alcohol sensor 17 acquired by the sensor value acquisition part 181 (step S105: YES), the measuring part 183 stores in an internal memory, the sensor value acquired by the sensor value acquisition part 181 in step S104 directly before the change in the sensor value occurred (step S106). Next, the timing part 182 begins measuring the elapsed time from the time at which the delivery of the breath occurred (step S107). Then, the sensor value acquisition part 181 acquires the sensor value of the semiconductor alcohol sensor 17 from the electrical signal output by the semiconductor alcohol sensor 17 (step S108). Next, the measuring part 183 determines whether or not the elapsed time from the time at which the delivery of the breath occurred measured by the timing part 182 has reached a predetermined measurement time (step S 109). If the measuring part 183 determines that the elapsed time has not reached the measurement time (step S109: NO), the process returns to step S108 to continue to acquire the sensor value.

On the other hand, if the measuring part 183 determines that the elapsed time has reached the measurement time (step S109: YES), the measuring part 183 stores in an internal memory, the sensor value acquired by the sensor value acquisition part 181 in step S108 directly before the measurement time was reached (step S110). Then, the measuring part 183 calculates the alcohol concentration contained in the breath based on the difference between the sensor value recorded in step S106 before the delivery of the breath, and the sensor value recorded in step S110 directly before the measurement time was reached (step S111).

Once the measuring part 183 has measured the alcohol concentration, the notification part 184 displays the alcohol concentration on the display 13 as shown in FIG. 4(C) (step S112). Next, the timing part 182 begins measuring the elapsed time from the time at which the alcohol concentration measurement was completed by the measuring part 183 (step S 113). The notification part 184 determines whether or not the elapsed time measured by the timing part 182 has reached a predetermined ventilation time (step S114).

If the notification part 184 determines that the elapsed time measured by the timing part 182 has not reached the ventilation time (ventilation period) (step S114: NO), the sensor value acquisition part 181 acquires a sensor value from the electrical signal output by the open/close sensor 15 (step S 115). The notification part 184 determines whether or not the sensor value acquired by the sensor value acquisition part 181 represents a releasing of the lid part 20 (step S116). If the notification part 184 determines that the sensor value of the open/close sensor 15 represents a releasing of the lid part 20 (step S116: YES), the process returns to step S114 to continue to determine whether or not the elapsed time has reached the ventilation time. At this time, the notification part 184 notifies the test subject that the lid part 20 is not to be closed owing to a ventilation of the interior of the housing, by displaying the word "CLEAN" on the display 13 as shown in FIG. 4(D), until the elapsed time reaches the ventilation time.

On the other hand, if the notification part 184 determines that the elapsed time has not reached the ventilation time, and the sensor value of the open/close sensor 15 indicates that the lid part 20 has been closed (step S 116: NO), the notification part 184 issues a notification prompting exposure of the semiconductor alcohol sensor 17 to the outside air, by scroll-displaying the word "OPEN" on the display 13 as shown in FIGS. 4(E) and 4(F), and further outputting an alarm from the speaker 14 (step S117). At this time, the timing part 182 stops measuring the elapsed time that was started in step S113.

Next, the timing part 182 begins measuring the elapsed time from the time at which the lid part 20 was closed (step S118). Then, the sensor value acquisition part 181 acquires the sensor value from the electrical signal output by the open/close sensor 15 (step S 119). The notification part 184 determines whether or not the sensor value acquired by the sensor value acquisition part 181 indicates a releasing of the lid part 20 (step S120). If the notification part 184 determines that the sensor value of the open/close sensor 15 indicates that the lid part 20 has been closed (step S120: NO), the notification part 184 determines whether or not the elapsed time measured by the timing part 182 has reached a predetermined notification stopping time (step S121). If the elapsed time has not reached the notification stopping time (step S121: NO), the process returns to step S119 and the notification part 184 continuously issues a notification prompting exposure of the semiconductor alcohol sensor 17 to the outside air. On the other hand, if the elapsed time has reached the notification stopping time (step S121: YES), the notification is stopped in order to suppress power consumption of the display 13 and the speaker 14, and the power of the alcohol concentration measurement apparatus 1 is turned off. Consequently, even if the test subject ignores the notification and leaves the lid part 20 closed, it becomes possible to prevent all of the power from being consumed before the next use.

On the other hand, in step S120, if the notification part 184 determines that the sensor value of the open/close sensor 15 indicates that the lid part 20 has been released (step S120: YES), the notification part 184 stops the notification prompting exposure of the semiconductor alcohol sensor 17 to the outside air. Next, the timing part 182 restarts measuring the elapsed time that was started in step S 113 (step S122).

Then the process returns to step S114 and continues to determine whether or not the elapsed time has reached the ventilation time. In step S122, the elapsed time is reset to 0 seconds. For example, the timing part 182 is configured to determine whether or not an elapsed time of the notification has reached a predetermined notification stopping time.

On the other hand, in step S 114, if the notification part 184 determines that the elapsed time measured by the timing part 182 has reached the ventilation time (step S114: YES), the power of the alcohol concentration measurement apparatus 1 is turned off, and processing is completed. Consequently, even if the lid part 20 is closed until the next time the power of the alcohol concentration measurement apparatus 1 is turned on, the notification part 184 does not issue a notification prompting exposure of the semiconductor alcohol sensor 17 to the outside air.

In this manner, according to the present embodiment, if the semiconductor alcohol sensor 17 is isolated from the outside air while the elapsed time from the time at which the alcohol concentration was measured is less than a predetermined ventilation time, the measuring part 183 issues a notification prompting exposure of the semiconductor alcohol sensor 17 to the outside air.

Consequently, it is possible to prevent the semiconductor alcohol sensor 17 from degradation due to a poisoning substance being confined in the interior of the housing 10.

### <Second Embodiment>

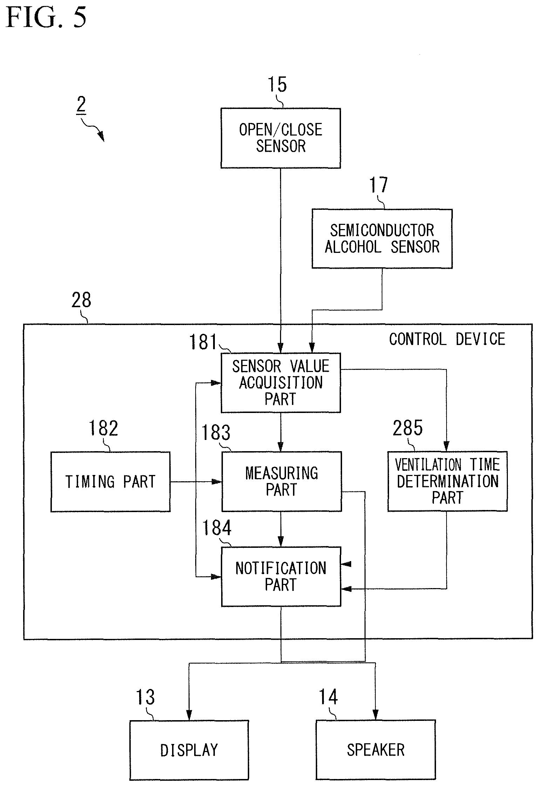

FIG. 5 is a schematic block diagram showing a configuration of an alcohol concentration measurement apparatus 2 according to a second embodiment.

The alcohol concentration measurement apparatus 2 according to the second embodiment is one in which, in addition to the configuration of the first embodiment, the control device 18 is further provided with a ventilation time determination part 285. Components of the second embodiment that are the same as those of the first embodiment are described using the same reference symbols as in the first embodiment.

The ventilation time determination part 285 determines, based on the alcohol concentration measured by the measuring part 183, a ventilation time for which the interior of the housing 10 is to be ventilated. For example, the ventilation time determination part 285 specifies the ventilation time according to a table that associates the concentration and the ventilation time. In the table, the ventilation time is a monotonically non-decreasing time with respect to the detected alcohol concentration. Consequently, the higher the probability of a breath containing a poisoning substance, the longer the ventilation time is made.

### <Third Embodiment>

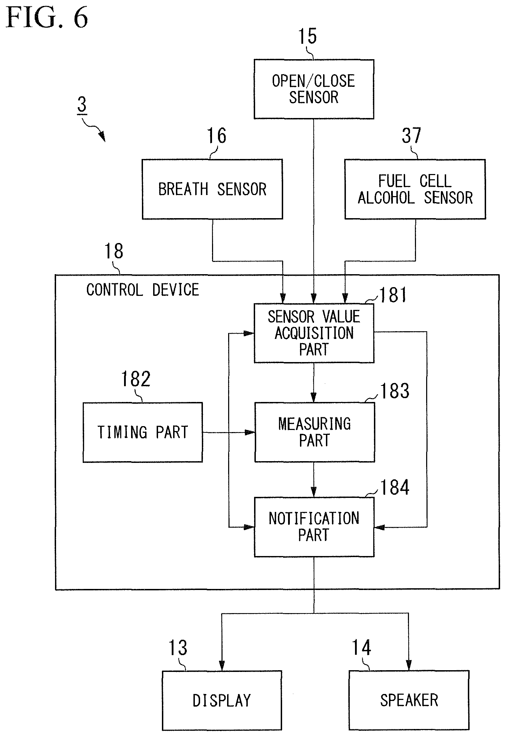

FIG. 6 is a schematic block diagram showing a configuration of an alcohol concentration measurement apparatus 3 according to a third embodiment.

The alcohol concentration measurement apparatus 3 according to the third embodiment is one that is provided with a fuel cell alcohol sensor 37 in place of the semiconductor alcohol sensor 17 of the first embodiment, and is further provided with a breath sensor 16. Components of the third embodiment that are the same as those of the first embodiment are described using the same reference symbols as in the first embodiment.

The breath sensor 16 detects the delivery of a breath through the ventilation holes 12, and outputs an electrical signal that indicates whether or not the delivery of a breath has occurred. The breath sensor 16, for example, can be realized by a pressure sensor that converts the pressure within the housing 10 into an electrical signal, or a flow sensor that converts the flow rate of the gas that flows through the vent holes 12 into an electrical signal.

The fuel cell alcohol sensor 37 is provided with a polymer membrane that is permeable to hydrogen ions, and a platinum catalyst. The platinum catalyst separates hydrogen ions from the alcohol in a breath, and the fuel cell alcohol sensor 37 generates electricity as a result of the hydrogen ions reacting with the oxygen in air via the polymer membrane. Consequently, the higher the alcohol concentration in a breath, the greater the quantity of hydrogen ions separated by the platinum catalyst, and thus the higher the electrical power generated by the fuel cell alcohol sensor 37.

On the other hand, if the fuel cell alcohol sensor 37 is exposed to the outside air or the breath for an extended period, the activation of the platinum catalyst gradually decreases. Consequently, the reaction rate of oxygen and hydrogen in the fuel cell alcohol sensor 37 becomes slower. That is to say, degradation of the semiconductor alcohol sensor 17 occurs. In the present embodiment, the semiconductor alcohol sensor 17 is an example of a gas sensor.

Next, operation of the alcohol concentration measurement apparatus 3 according to the third embodiment is described.

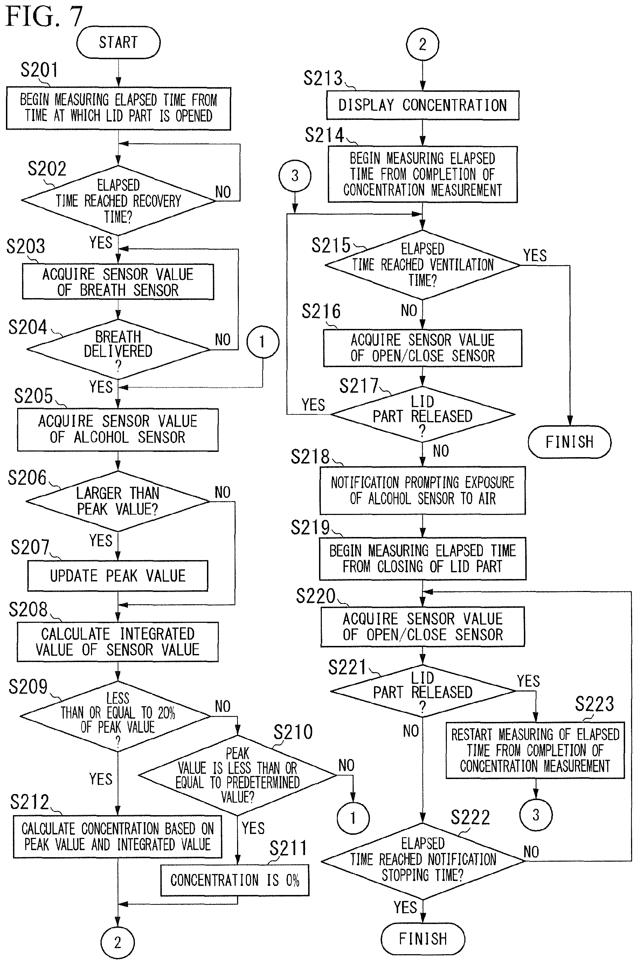

FIG. 7 is a flowchart showing operation of the alcohol concentration measurement apparatus 3 according to the third embodiment.

When the sensor value acquisition part 181 acquires from the open/close sensor 15 the electrical signal that indicates the releasing of the lid part 20, the timing part 182 begins measuring the elapsed time from the time at which the lid part 20 is opened (step S201). The measuring part 183 determines whether or not the elapsed time measured by the timing part 182 has reached a predetermined recovery time (for example, 10 seconds) (step S202). The recovery time represents a time for removing the alcohol deposited on the fuel cell alcohol sensor 37 from the fuel cell alcohol sensor 37 in the previous measurement. If the elapsed time measured by the timing part 182 has not reached the recovery time (step S202: NO), the process returns to step S202, and repeats the determination of whether or not the elapsed time has reached the recovery time. The alcohol concentration measurement apparatus 3 accepts the delivery of a breath after the elapsed time measured by the timing part 182 reaches the predetermined recovery time. That is to say, even if delivery of a breath occurs before the recovery time has passed, the measuring part 183 does not perform an alcohol concentration measurement. Furthermore, until the elapsed time measured by the timing part 182 reaches the recovery time, the notification part 184 displays on the display 13 as shown in FIG. 4(A), the time remaining until the elapsed time reaches the recovery time.

On the other hand, if the measuring part 183 determines that the elapsed time measured by the timing part 182 has reached the recovery time (step S202: YES), the sensor value acquisition part 181 acquires a sensor value from the electrical signal output by the breath sensor 16 (step S203). The measuring part 183 determines whether or not the sensor value of the breath sensor 16 acquired by the sensor value acquisition part 181 indicates the delivery of a breath (whether a breath is delivered/blown or not) (step S204). If the measuring part 183 determines that the sensor value of the breath sensor 16 indicates that a breath has not been delivered (step S204: NO), the process returns to step S203 and awaits the delivery of a breath. Until the delivery of a breath is detected, the notification part 184 prompts the test subject to deliver a breath by displaying the word "START" on the display 13 as shown in FIG. 4(B).

If the measuring part 183 determines that the sensor value of the breath sensor 16 acquired by the sensor value acquisition part 181 indicates that the delivery of a breath occurred (step S204: YES), the sensor value acquisition part 181 acquires a sensor value of the fuel cell alcohol sensor 37 from an electrical signal output by the fuel cell alcohol sensor 37 (step S205). The measuring part 183 determines whether or not the sensor value of the fuel cell alcohol sensor 37 acquired by the sensor value acquisition part 181 is larger than a peak value of the sensor values acquired after the time at which the delivery of the breath was detected in step S204 (step S206). If the measuring part 183 determines that the acquired sensor value is larger than the peak value (step S206: YES), the measuring part 183 updates the peak value (step S207). On the other hand, if the measuring part 183 determines that the acquired sensor value is less than or equal to the peak value (step S206: NO), the measuring part 183 does not update the peak value. Then, the measuring part 183 calculates an integrated value of the sensor value of the fuel cell alcohol sensor 37 (step S208).

Next, the measuring part 183 determines whether or not the sensor value of the fuel cell alcohol sensor 37 is less than or equal to 20% of the peak value (step S209). If the measuring part 183 determines that the sensor value of the fuel cell alcohol sensor 37 is greater than 20% of the peak value (step S209: NO), the measuring part 183 determines whether or not a peak value measured within a predetermined time (for example, 1 second) from the starting of the acquisition of the sensor values of the fuel cell alcohol sensor 37 is less than or equal to a predetermined threshold (step S210).

If the measuring part 183 determines that the sensor value of the fuel cell alcohol sensor 37 is larger than 20% of the peak value, and that the peak value is larger than the threshold (step S210: NO), the process returns to step S205 to continue the acquisition of sensor values of the fuel cell alcohol sensor 37. On the other hand, if the measuring part 183 determines that the value of the acquired signal is larger than 20% of the peak value, and that the peak value is larger than the threshold (step S210: YES), the measuring part 183 determines that the breath does not contain alcohol (step S211). That is to say, the measuring part 183 determines that the alcohol concentration contained in the breath is 0%.

On the other hand, if the measuring part 183 determines in step S209 that the sensor value of the fuel cell alcohol sensor 37 is less than or equal to 20% of the peak value (step S209: YES), the measuring part 183 measures the alcohol concentration contained in the breath based on the peak value and the integrated value of the sensor value (step S212).

Once the measuring part 183 has measured the alcohol concentration in step S211 or step S212, the notification part 184 displays the alcohol concentration on the display 13 as shown in FIG. 4(C) (step S213). Next, the timing part 182 begins measuring the elapsed time from the time at which the alcohol concentration measurement was completed by the measuring part 183 (step S214). The notification part 184 determines whether or not the elapsed time measured by the timing part 182 has reached a predetermined ventilation time (step S215).

If the notification part 184 determines that the elapsed time measured by the timing part 182 has not reached the ventilation time (step S215: NO), the sensor value acquisition part 181 acquires the sensor value from the electronic signal output by the open/close sensor 15 (step S216). The notification part 184 determines whether or not the sensor value acquired by the sensor value acquisition part 181 represents a releasing of the lid part 20 (step S217). If the notification part 184 determines that the sensor value of the open/close sensor 15 represents a releasing of the lid part 20 (step S217: YES), the process returns to step S215 to continue to determine whether or not the elapsed time has reached the ventilation time. At this time, the notification part 184 notifies the test subject that the lid part 20 is not to be closed owing to a ventilation of the interior (interior air) of the housing 10, by displaying the word "CLEAN" on the display 13 as shown in FIG. 4(D), until the elapsed time reaches the ventilation time.

On the other hand, if the notification part 184 determines that the elapsed time has not reached the ventilation time, and the sensor value of the open/close sensor 15 indicates that the lid part 20 has been closed (step S217: NO), the notification part 184 issues a notification prompting exposure of the fuel cell alcohol sensor 37 to the outside air, by scroll-displaying the word "OPEN" on the display 13 as shown in FIG. 4(E) and 4(F), and further outputting an alarm from the speaker 14 (step S218). At this time, the timing part 182 stops measuring the elapsed time that was started in step S214.

Next, the timing part 182 begins measuring the elapsed time from the time at which the lid part 20 has been closed (step S219). Then, the sensor value acquisition part 181 acquires the sensor value from the electrical signal output by the open/close sensor 15 (step S220). The notification part 184 determines whether or not the sensor value acquired by the sensor value acquisition part 181 indicates a releasing of the lid part 20 (step S221). If the notification part 184 determines that the sensor value of the open/close sensor 15 indicates that the lid part 20 has been closed (step S221: NO), the notification part 184 determines whether or not the elapsed time measured by the timing part 182 has reached a predetermined notification stopping time (step S222). If the elapsed time has not reached the notification stopping time (step S222: NO), the process returns to step S220 and the notification part 184 continuously issues a notification prompting exposure of the fuel cell alcohol sensor 37 to the outside air. On the other hand, if the elapsed time has reached the notification stopping time (step S222: YES), the notification is stopped in order to suppress power consumption of the display 13 and the speaker 14, and the power of the alcohol concentration measurement apparatus 3 is turned off. Consequently, even if the test subject ignores the notification and leaves the lid part 20 closed, it becomes possible to prevent all of the power from being consumed before the next use.

On the other hand, in step S221, if the notification part 184 determines that the sensor value of the open/close sensor 15 indicates that the lid part 20 has been released (step S221: YES), the notification part 184 stops the notification prompting exposure of the fuel cell alcohol sensor 37 to the outside air. Next, the timing part 182 restarts measuring the elapsed time that was started in step S214 (step S223). Then, the process returns to step S215 and continues to determine whether or not the elapsed time has reached the ventilation time. In step S223, the elapsed time is reset to 0 seconds. For example, the timing part 182 is configured to determine whether or not an elapsed time of the notification has reached a predetermined notification stopping time.

On the other hand, in step S215, if the notification part 184 determines that the elapsed time measured by the timing part 182 has reached the ventilation time (step S215: YES), the power of the alcohol concentration measurement apparatus 3 is turned off, and processing is completed. Consequently, even if the lid part 20 is closed until the next time the power of the alcohol concentration measurement apparatus 3 is turned on, the notification part 184 does not issue a notification prompting exposure of the fuel cell alcohol sensor 37 to the outside air.

The foregoing has described in detail several embodiments with reference to the drawings. However specific configurations are in no way limited to those mentioned above, and various design changes, and the like, are possible.

For example, in the embodiment described above, a case is described where the power is turned off in order to suppress power consumption if, after issuing a notification prompting exposure of the gas sensor to the outside air, the notification stopping time passes without the lid part 20 being released. However, it is in no way limited to this. For example, in another embodiment, the notification part 184 may continue the notification irrespective of the elapsed time from the time at which the notification prompting exposure of the gas sensor to the outside air was first issued. Consequently, it becomes possible to more firmly prompt the test subject to expose the gas sensor to the outside air. Furthermore, if as a consequence, electric power necessary for start-up the next time does not remain, that fact allows the test subject to recognize that it is necessary to leave the lid part 20 released for a period equivalent to the ventilation time. Moreover, for example, in another embodiment, the notification part 184 may issue a notification prompting exposure of the gas sensor to the outside air at predetermined intervals.

Furthermore, in the embodiment mentioned above, a case is described where the gas concentration measurement apparatus is realized by the alcohol concentration measurement apparatuses 1, 2 and 3. However, it is in no way limited to this. For example, the functions of the gas concentration measurement apparatus according to the present invention may be applied to other gas concentration measurement apparatuses. Examples of other gas concentration measurement apparatuses include a body fat burn amount measuring apparatus that measures the extent to which body fat is burned by detecting the acetone concentration in breath (a concentration of a gas in breath that indicates a body fat burn amount), and an oral odor detection apparatus that measures the concentration of gases that cause oral odor, such as methylmercaptan. The gas sensor (biological gas sensor) of the gas concentration measurement apparatus (biological gas concentration measurement apparatus) can be configured to detect at least one of alcohol in breath, a gas in breath as a marker for body fat burning and a gas in breath that causes oral odor).

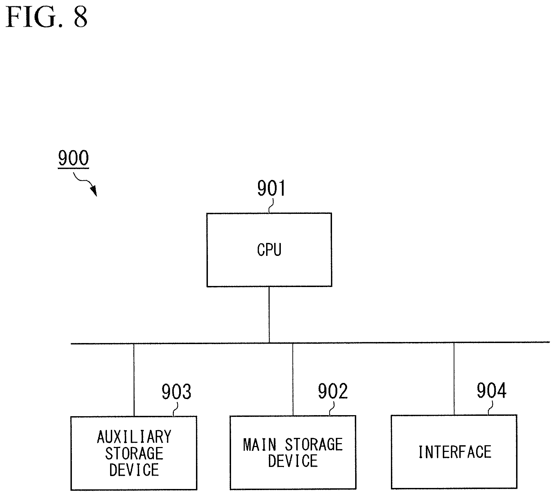

FIG. 8 is a schematic block diagram showing a configuration of a computer 900 according to at least one embodiment.

The computer 900 is provided with a CPU 901, a main storage device 902, an auxiliary storage device 903, and an interface 904.

The control devices 18 and 28 described above are provided with the computer 900. Further, the operations of the processing parts mentioned above are stored in the form of a program in the auxiliary storage device 903. The CPU 901 reads the program from the auxiliary storage device 903 and transfers the program to the main storage device 902, and the processing mentioned above is executed according to the program.

In at least one embodiment, the auxiliary storage device 903 is an example of a non-transitory tangible medium. Examples of other non-transitory tangible media include a magnetic disk, a magnet-optical disk, a CD-ROM, a DVD-ROM, and a semiconductor memory that are connected via the interface 904. Furthermore, if the program is transmitted to the computer 900 by a transmission line, the computer 900 that receives the transmission may transfer the program to the main storage device 902, and execute the processing mentioned above.

Moreover, the program may be one that realizes a portion of the functions mentioned above.

Further, the program may be one that realizes the functions mentioned above by being combined with another program that has previously been stored in the auxiliary storage device 903, as a so-called difference file (difference program).

## Claims

1. A gas concentration measurement apparatus (1) comprising:
a main body (10);
a gas sensor (17);
a member (20) arranged so that the gas sensor is isolated from the outside air when the member is arranged at a closing position and that the gas sensor is exposed to the outside air when the member is arranged at an opening position;
a measuring part (183) configured to measure a concentration of a gas on the basis of an output signal from the gas sensor; and
a timing part (182) configured to measure an elapsed time from a time at which the concentration of the gas is measured by the measuring part;
**characterized by**
a notification part (184) configured to, in a case where the gas sensor is isolated from outside air while the measured elapsed time is less than a predetermined ventilation time that is required to ventilate the gas sensor, issue a notification prompting exposure of the gas sensor to the outside air.

2. The gas concentration measurement apparatus according to Claim 1, wherein
the timing part stops measuring the elapsed time in a case where the gas sensor is isolated from outside air while the measured elapsed time is less than the predetermined ventilation time, and restarts measuring the elapsed time in a case where the gas sensor is exposed to the outside air.

3. The gas concentration measurement apparatus according to Claim 1 or 2, wherein
the timing part is configured to determine whether or not an elapsed time of the notification has reached a predetermined notification stopping time.

4. The gas concentration measurement apparatus according to any one of Claims 1 to 3, further comprising:
a ventilation time determination part configured to determine a ventilation time based on the concentration of the gas measured by the measuring part.

5. The gas concentration measurement apparatus according to any one of Claims 1 to 4, wherein
the member is configured to be slidable with respect to a part of the main body, and
the member is arranged to be prevented from detachment from the main body.

6. The gas concentration measurement apparatus according to any one of Claims 1 to 5, wherein
the member is configured to be slidable with respect to a part of the main body, and
wherein the main body is provided with a metallic frame.

7. The gas concentration measurement apparatus according to any one of Claims 1 to 6, further comprising:
an open/close sensor (15) configured to detect whether the member is at the closing position or not.

8. The gas concentration measurement apparatus according to any one of Claims 1 to 7, wherein
the gas sensor is configured to measure an alcohol concentration in breath.

9. The gas concentration measurement apparatus according to any one of Claims 1 to 7, wherein
the gas sensor is configured to measure at least one of a concentration of a gas in breath that indicates a body fat burn amount and a gas in breath that causes oral odor.

10. A notification method for a gas concentration measurement apparatus (1) including:
a main body (10);
a gas sensor (17); and
a member (20) arranged so that the gas sensor is isolated from the outside air when the member is arranged at a closing position and that the gas sensor is exposed to the outside air when the member is arranged at an opening position;
the method comprising:
measuring a concentration of a gas on the basis of an output signal from the gas sensor (S203-S212); and
measuring an elapsed time from a time at which the concentration of the gas is measured (S214);
**characterized in that**
in a case where the gas sensor is isolated from outside air while the measured elapsed time is less than a predetermined ventilation time that is required to ventilate the gas sensor, issuing a notification prompting exposure of the gas sensor to the outside air (S218).

11. The notification method according to Claim 11, further comprising:
in a case where the gas sensor is isolated from outside air while the measured elapsed time is less than the predetermined ventilation time, stopping the measurement of the elapsed time (S222); and
restarting the measurement of the elapsed time in a case where the gas sensor is exposed to the outside air (S223).

12. The notification method according to Claim 10 or 11, further comprising:
determining whether or not an elapsed time of the notification has reached a predetermined notification stopping time (S222).

13. The notification method according to any one of Claims 10 to 12, further comprising:
determining the ventilation time based on the measured concentration of the gas.

14. A computer-readable storage medium stored with a notification program for a gas concentration measuring apparatus including:
a main body (10);
a gas sensor (17); and
a member (20) arranged so that the gas sensor is isolated from the outside air when the member is arranged at a closing position and that the gas sensor is exposed to the outside air when the member is arranged at an opening position;
the notification program being configured to cause a computer to:
measure a concentration of a gas on the basis of an output signal from the gas sensor (S203-S212);
measure an elapsed time from a time at which the concentration of the gas is measured (S214); and
in a case where the gas sensor is isolated from outside air while the measured elapsed time is less than a predetermined ventilation time that is required to ventilate the gas sensor, issues a notification prompting exposure of the gas sensor to the outside air (S218).

## Patentansprüche

1. Gaskonzentrationsmessvorrichtung (1) umfassend:
einen Hauptkörper (10);
einen Gassensor (17);
ein Element (20) angeordnet, so dass der Gassensor von der Außenluft isoliert ist, wenn das Element in einer Schließposition angeordnet ist und dass der Gassensor der Außenluft ausgesetzt ist, wenn das Element in einer Öffnungsposition angeordnet ist;
ein Messteil (183), welches konfiguriert ist, um eine Konzentration eines Gases auf der Basis eines Ausgangssignals von dem Gassensor zu messen; und
ein Timing-Teil (182), welches konfiguriert ist, um eine von einem Zeitpunkt, zu welchem die Konzentration des Gases von dem Messteil gemessen wird, verstrichene Zeit zu messen;
**gekennzeichnet durch**
ein Benachrichtigungsteil (184), welches konfiguriert ist, um in einem Fall, in dem der Gassensor von der Außenluft isoliert ist, während die gemessene verstrichene Zeit geringer als eine vorbestimmte, zur Belüftung des Gassensors notwendige Belüftungszeit ist, eine Benachrichtigung auszugeben, den Gassensor der Außenluft auszusetzen.

2. Gaskonzentrationsrüessvorrichtung nach Anspruch 1, wobei das Timing-Teil das Messen der verstrichenen Zeit in einem Fall beendet, in dem der Gassensor von der Außenluft isoliert ist, während die gemessene verstrichene Zeit geringer ist als die vorbestimmte Belüftungszeit und das Messen der verstrichenen Zeit in einem Fall neustartet, in dem der Gassensor der Außenluft ausgesetzt ist.

3. Gaskonzentrationsmessvorrichtung nach Anspruch 1 oder 2, wobei das Timing-Teil konfiguriert ist, um zu bestimmen ob eine verstrichene Zeit der Benachrichtigung eine vorbestimmte Benachrichtigungsanhaltezeit erzielt hat.

4. Gaskonzentrationsmessvorrichtung nach einem der Ansprüche 1 bis 3, des Weiteren umfassen:
ein Belüftungszeitbestimmungsteil, welcher konfiguriert ist, um eine Belüftungszeit basierend auf der Konzentration des von dem Messteil gemessenen Gases zu bestimmen.

5. Gaskonzentrationsmessvorrichtung nach einem der Ansprüche 1 bis 4, wobei das Element in Bezug auf einen Teil des Hauptkörpers verschiebbar konfiguriert ist und das Element angeordnet ist, um eine Entfernung von dem Hauptkörper zu verhindern.

6. Gaskonzentrationsmessvorrichtung nach einem der Ansprüche 1 bis 5, wobei das Element in Bezug auf einen Teil des Hauptkörpers verschiebbar konfiguriert ist und wobei der Hauptkörper mit einem metallischen Rahmen bereitgestellt ist.

7. Gaskonzentrationsmessvorrichtung nach einem der Ansprüche 1 bis 6, des Weiteren umfassend: einen Öffnungs/Schließsensor (15), welcher konfiguriert ist, um zu ermitteln, ob sich das Element in der Schließposition befindet oder nicht.

8. Gaskonzentrationsmessvorrichtung nach einem der Ansprüche 1 bis 7, wobei der Gassensor konfiguriert ist, um eine Alkoholkonzentration in einer Atemluft zu messen.

9. Gaskonzentrationsmessvorrichtung nach einem der Ansprüche 1 bis 7, wobei der Gassensor konfiguriert ist, um wenigstens eine aus einer Konzentration eines Gases in einer Atemluft, das eine Körperfettverbrennungsmenge angibt, und eines Gases in der Atemluft, das Mundgeruch verursacht, zu messen.

10. Benachrichtigungsverfahren für eine Gaskonzentrationsmessvorrichtung (1) umfassend:
einen Hauptkörper (10);
einen Gassensor (17); und
ein Element (20), welches angeordnet ist, so dass der Gassensor von der Außenluft isoliert ist, wenn das Element in einer Schließposition angeordnet ist und dass der Gassensor der Außenluft ausgesetzt ist, wenn das Element in einer Öffnungsposition angeordnet ist;
wobei das Verfahren umfasst:
Messen einer Konzentration eines Gases auf der Basis eines Ausgangssignals von dem Gassensor (S203-S212); und
Messen einer von einem Zeitpunkt, zu welchem die Konzentration des Gases von dem Messteil gemessen wird, verstrichenen Zeit (S214);
**dadurch gekennzeichnet, dass**
in einem Fall, in dem der Gassensor von der Außenluft isoliert ist, während die gemessene verstrichene Zeit geringer als eine vorbestimmte, zur Belüftung des Gassensors notwendige Belüftungszeit ist, Ausgeben einer Benachrichtigung den Gassensor der Außenluft auszusetzen.

11. Benachrichtigungsverfahren nach Anspruch 11, des Weiteren umfassend:
in einem Fall, in dem der Gassensor von der Außenluft isoliert ist, während die gemessene verstrichene Zeit geringer als die vorbestimmte Belüftungsdauer ist, Beenden der Messung der verstrichenen Zeit (S222); und
Neustarten der Messung der verstrichenen Zeit in einem Fall, in dem der Gassensor der Außenluft ausgesetzt ist (S223).

12. Benachrichtigungsverfahren nach Anspruch 10 oder 11, des Weiteren umfassend: Bestimmen, ob eine verstrichene Zeit der Benachrichtigung eine vorbestimmte Benachrichtigungsanhaltezeit (S222) erreicht hat oder nicht.

13. Benachrichtigungsverfahren nach einem der Ansprüche 10 bis 12, des Weiteren umfassend: Bestimmen der Belüftungszeit basierend auf der gemessenen Konzentration des Gases.

14. Computer lesbares Speichermedium, mit einem gespeicherten Benachrichtigungsprogramm für eine Gaskonzentrationsmessvorrichtung umfassend:
einen Hauptkörper (10);
einen Gassensor (17); und
ein Element (20), welches angeordnet ist, so dass der Gassensor von der Außenluft isoliert ist, wenn das Element in einer Schließposition angeordnet ist und dass der Gassensor der Außenluft ausgesetzt ist, wenn das Element in einer Öffnungsposition angeordnet ist;
wobei das Verfahren umfasst:
Messen einer Konzentration eines Gases auf der Basis eines Ausgangssignals von dem Gassensor (S203-S212); und
Messen einer von einem Zeitpunkt, zu welchem die Konzentration des Gases von dem Messteil gemessen wird, verstrichenen Zeit (S214);
**dadurch gekennzeichnet, dass**
in einem Fall, in dem der Gassensor von der Außenluft isoliert ist, während die gemessene verstrichene Zeit geringer als eine vorbestimmte, zur Belüftung des Gassensors notwendige Belüftungszeit ist, Ausgeben einer Benachrichtigung den Gassensor der Außenluft auszusetzen.

## Revendications

1. Appareil de mesure de la concentration de gaz (1) comprenant:
un corps principal (10);
un capteur de gaz (17) ;
un élément (20) agencé de sorte que le capteur de gaz est isolé de l'air extérieur lorsque l'élément occupe une position de fermeture et que le capteur de gaz est exposé à l'air extérieur lorsque l'élément occupe une position d'ouverture;
une partie mesure (183) configurée pour mesurer une concentration d'un gaz en fonction d'un signal de sortie provenant du capteur de gaz; et
une partie synchronisation (182) configurée pour mesurer un temps écoulé depuis un instant auquel la concentration du gaz est mesurée par la partie mesure;
**caractérisé par**:
une partie notification (184) configurée pour, au cas où le capteur de gaz est isolé de l'air extérieur tandis que le temps écoulé mesuré est inférieur à un temps de ventilation prédéterminé qui est nécessaire pour ventiler le capteur de gaz, émettre une notification demandant l'exposition du capteur de gaz à l'air extérieur.

2. Appareil de mesure de la concentration de gaz selon la revendication 1, dans lequel
la partie synchronisation arrête la mesure du temps écoulé au où le capteur de gaz est isolé de l'air extérieur tandis que le temps écoulé mesuré est inférieur à la durée de ventilation prédéterminée, et redémarre la mesure de la durée écoulée au cas où le capteur de gaz est exposé à l'air extérieur.

3. Appareil de mesure de la concentration de gaz selon la revendication 1 ou la revendication 2, dans lequel
la partie synchronisation est configurée pour déterminer si oui ou non un temps écoulé de la notification a atteint un temps d'arrêt de notification prédéterminé.

4. Appareil de mesure de la concentration de gaz selon l'une quelconque des revendications 1 à 3, comprenant en outre:
une partie détermination du temps de ventilation configurée pour déterminer un temps de ventilation en fonction de la concentration du gaz mesurée par la partie mesure.

5. Appareil de mesure de la concentration de gaz selon l'une quelconque des revendications 1 à 4, dans lequel
l'élément est configuré de manière à pouvoir coulisser par rapport à une partie du corps principal, et
l'élément est agencé pour ne pas se détacher du corps principal.

6. Appareil de mesure de la concentration de gaz selon l'une quelconque des revendications 1 à 5, dans lequel
l'élément est configuré de manière à pouvoir coulisser par rapport à une partie du corps principal, et dans lequel
le corps principal est pourvu d'un cadre métallique.

7. Appareil de mesure de la concentration de gaz selon l'une quelconque des revendications 1 à 6, comprenant en outre:
un capteur d'ouverture/fermeture (15) configuré pour détecter si l'élément occupe la position de fermeture ou non.

8. Appareil de mesure de la concentration de gaz selon l'une quelconque des revendications 1 à 7, dans lequel
le capteur de gaz est configuré pour mesurer une concentration d'alcool dans la respiration.

9. Appareil de mesure de la concentration de gaz selon l'une quelconque des revendications 1 à 7, dans lequel
le capteur de gaz est configuré pour mesurer au moins la concentration d'un gaz dans la respiration qui indique une quantité de combustion de la graisse corporelle et/ou d'un gaz dans la respiration qui provoque une mauvaise haleine.

10. Procédé de notification pour un appareil de mesure de la concentration de gaz (1) incluant:
un corps principal (10);
un capteur de gaz (17); et
un élément (20) agencé de sorte que le capteur de gaz est isolé de l'air extérieur lorsque l'élément occupe une position de fermeture et que le capteur de gaz est exposé à l'air extérieur lorsque l'élément occupe une position d'ouverture;
le procédé comprenant:
la mesure d'une concentration d'un gaz en fonction d'un signal de sortie provenant du capteur de gaz (S203-S212); et
la mesure d'un temps écoulé depuis un instant auquel la concentration du gaz est mesurée (S214);
**caractérisé en ce que**,
au cas où le capteur de gaz est isolé de l'air extérieur tandis que le temps écoulé mesuré est inférieur à un temps de ventilation prédéterminé qui est nécessaire pour ventiler le capteur de gaz, une notification est émise demandant l'exposition du capteur de gaz à l'air extérieur (S218).

11. Procédé selon la revendication 11, comprenant en outre:
au cas où le capteur de gaz est isolé de l'air extérieur tandis que le temps écoulé mesuré est inférieur à la durée de ventilation prédéterminée, l'arrêt de la mesure du temps écoulé (S222); et
le redémarrage de la mesure du temps écoulé au cas où le capteur de gaz est exposé à l'air extérieur (S223).

12. Procédé selon la revendication 10 ou la revendication 11, consistant en outre:
à déterminer si oui ou non un délai écoulé de la notification a atteint un temps d'arrêt de notification prédéterminé (S222).

13. Procédé de notification selon l'une quelconque des revendications 10 à 12, comprenant en outre:
la détermination du temps de ventilation en fonction de la concentration mesurée du gaz.

14. Support de stockage lisible par ordinateur stocké avec un programme de notification pour un appareil de mesure de la concentration de gaz incluant:
un corps principal (10);
un capteur de gaz (17); et
un élément (20) agencé de sorte que le capteur de gaz est isolé de l'air extérieur lorsque l'élément occupe une position de fermeture et que le capteur de gaz est exposé à l'air extérieur lorsque l'élément occupe une position d'ouverture;
le programme de notification étant configuré pour amener un ordinateur à:
mesurer une concentration d'un gaz en fonction d'un signal de sortie provenant du capteur de gaz (S203-S212);
mesurer un temps écoulé depuis un instant auquel la concentration du gaz est mesurée (S214); et
au cas où le capteur de gaz est isolé de l'air extérieur tandis que le temps écoulé mesuré est inférieur à un temps de ventilation prédéterminé qui est nécessaire pour ventiler le capteur de gaz, une notification est émise demandant l'exposition du capteur de gaz à l'air extérieur (S218).
